(19) Europäisches Patentamt
European Patent Office
Office européen des brevets

(11) Publication number: **0 457 908 A1**

(12)
# EUROPEAN PATENT APPLICATION published in accordance with Art. 158(3) EPC

(21) Application number: **90905365.4**

(22) Date of filing: **27.11.89**

(86) International application number:
**PCT/SU89/00302**

(87) International publication number:
**WO 91/07968 (13.06.91 91/13)**

(51) Int. Cl.[5]: **A61K 31/495**, A61K 9/20, C07D 487/04, C07D 209/86

(43) Date of publication of application:
**27.11.91 Bulletin 91/48**

(84) Designated Contracting States:
**BE CH DE FR GB IT LI SE**

(71) Applicant: **VSESOJUZNY NAUCHNO-ISSLEDOVATELSKY KHIMIKO-FARMATSEVTICHESKY INST. IMENI SERGO ORDZHONIKIDZE (VNIKHFI)**
**ul. Zubovskaya, 7**
**Moscow, 119815(SU)**

(72) Inventor: **SHVEDOV, Vasily Ivanovich**
**ul. M.Ulyanovoi, 9-3-11**
**Moscow, 117331(SU)**
Inventor: **MASHKOVSKY, Mikhail Davydovich**
**Leningradsky pr., 75a-55**
**Moscow, 125057(SU)**
Inventor: **SAVITSKAYA, Nina Viktorovna**
**ul. K.Marxa, 27/21-19 Moskovskaya obl.**
**Kaliningrad, 141070(SU)**
Inventor: **ANDREEVA, Natalia Ivanovna**
**Frunzenskaya nab., 38/1-14**
**Moscow, 119270(SU)**
Inventor: **FEDOROVA, Irina Nikolaevna**
**ul. G.Kurina, 4-4-55**
**Moscow, 121108(SU)**
Inventor: **GUSKOVA, Tatyana Anatolievna**
**ul. Sadovaya-Spasskaya, 12/23-19**
**Moscow, 103045(SU)**
Inventor: **TUPIKINA, Svetlana Mikhailovna**
**ul. Revoljutsii, 47 Moskovskaya obl.**
**Ramenskoe, 140100(SU)**
Inventor: **VERSTAKOVA, Olga Lvovna**
**Keramichesky proezd, 73-1-93**
**Moscow, 127591(SU)**
Inventor: **NYRKOVA, Valentina Georgievna**
**ul. Khersonskaya, 7-1-20**
**Moscow, 113461(SU)**
Inventor: **KONYAEVA, Irina Pavlovna**
**ul. Matveevskaya, 18-2-2**
**Moscow, 119517(SU)**
Inventor: **ALTUKHOVA, Ljudmila Benediktovna**
**Zagorodnoe shosse, 8-5-23**
**Moscow, 113152(SU)**
Inventor: **AKALAEVA, Tatyana Vasilievna**
**pos. Mechnikova, 17-38 Krasnogorsky raion**
**Moskovskaya obl., 143422(SU)**
Inventor: **ASNINA, Valentina Vasilievna**
**ul. Klyazminskaya, 32-1-27**
**Moscow, 127644(SU)**
Inventor: **GORKIN, Vladimir Zinovievich**
**Rostovskaya nab., 5-36**
**Moscow, 119121(SU)**
Inventor: **VEREVKINA, Iren Vladimirovna**
**ul. Matveevskaya, 10-2-53**
**Moscow, 119517(SU)**
Inventor: **GLUSHKOV, Robert Georgievich**
**ul. Gorkogo, 43-90**
**Moscow, 125047(SU)**
Inventor: **ARJUZINA, Vera Mikhailovna**
**ul. Matveevskaya, 10-4-60**
**Moscow, 119517(SU)**
Inventor: **EROFEEV, Jury Vladimirovich**
**Pribrezhny proezd, 3-118**
**Moscow, 125445(SU)**
Inventor: **GRINEV, Alexei Nikolaevich**

**deceased(SU)**

(74) Representative: **Spencer, Graham Easdale et al**

**A.A. Thornton & CO Northumberland House**
**303-306, High Holborn**
**London WC1V 7LE(GB)**

(54) **HYDROCHLORIDE 2,3,3a,4,5,6-HEXAHYDRO-8-CYCLOHEXYL-1H-PYRAZINO (3,2,1-j,k)CARBAZOL, METHOD OF OBTAINING IT AND A PHARMACEUTICAL PREPARATION OF ANTIDEPRESSIVE ACTION BASED ON IT.**

(57) Hydrochloride 2,3,3a,4,5,6-hexahydro-8-cyclohexyl-1H-pyrazino (3,2,1-j,k)carbazol has formula (I). A method of obtaining the claimed composition consists in that 6-cyclohexyl-3,4-dihydrocarbazol-1(2H) is alkylated by hydrochloride β-(N,N-dibenzylamino) ethylchloride in a two-phase system: an aromatic hydrocarbon-34-40 %-aqueous solution of alkali in the presence of an interphase transfer catalyst at a temperature of 85-110 °C with subsequent hydrogenation of the resulting 6-cyclohexyl-9β-(N,N-dibenzylamino) ethyl-3,4-dihydrocarbazol-1(2H) over palladium catalyst on a carrier in the medium of a higher aliphatic alcohol at a temperature of 50-70 °C and under a pressure of 1 to 30 atm with subsequent transfer of the obtained 2,3,3a,4,5,6-hexahydro-8-cyclohexyl-1H-pyrazino (3,2,1-j,k)carbazol into hydrochloride and extraction of the latter. The claimed composition has an antidepressive action and is an active substance of a pharmaceutical preparation with antidepressive action.

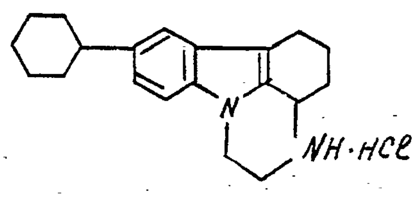

(I)

Field of the Invention

The present invention relates to the art of organic chemistry and, more particularly, to a method for preparing a novel compound - 2,3,3a,4,5,6-hexahydro-8-cyclohexyl--1H-pyrazino (3,2,1-j,k)carbazole hydrochloride, and to an antidepressive drug based thereon.

State of the Art

Known in the art are different compounds exhibiting an antidepressive effect such as N-(3-dimethylaminopropyl)-aminodibenzyl hydrochloride and pharmaceutical preparations based thereon, namely: melipramine (imipramine, tophranyl) (Arch.int.Pharmacodyn., 1964, 151, 1-2, p. 180-191). This drug releaves or lowers the depriming effects of reserpine, enhances the effect of sympathomimetic substances, exhibits a cholinolytic activity. As regards the character of its action, it pertains to antidepressants with an accompanying stimulating effect and is useful for the therapy of depressive states of various aetiology, especially in the case of astheno-depressive states with a pronounced motor and ideamotor retardation. Furthermore, administration of imipramine is limited (especially in elderly people) due to its cardiotoxicity and cholinolytic properties.

Also known in the art is another compound, namely: 2,3,3a,4,5,6-hexahydro-8-methyl-1H-pyrazino(3,2,1-j,k)-carbazole hydrochloride which is an active principle of a preparation pyrazidol (pyrlindol, Pirlindolum, Perlindole) (GB, A, 1340528).

Pyrazidol shortens the time of immobilization in emotion-stress behaviour tests, reduces depressive effects of reserpine, enhances the central effect of d,l-amphetamine, l-dofa. 5-hydroxytryptophane. Pyrazidol is a reversible, selective inhibitor of monoaminoxidase of the A type. In addition, it also suppresses a reverse capture of norepinephrine and serotonin by synaptosomes of rats' brain.

Pyrazidol does not provide any cholinolytic effect. The drug is effective in the case of depressions with a psychomotor retardation of a different etiology. The absence of the cholinolytic effect enables administration of the preparation where imipromine and other preparations of this group are contraindicated. However, in some cases administration of pyrazidol results in xerostomia, tachycardia, giddiness. The therapeutic effect of this drug manifests itself only by the 5-th - 12-th day.

Disclosure of the Invention

The present invention is novel and hitherto unknown in the literature.

The present invention is amed at providing a novel compound having a high antidepressive effect, featuring, rapid and broad therapeutical action, a low toxicity and a good tolerance by patients, and to the provision of a method for preparing this compound.

The problem is solved by a novel compound, viz. 2,3,3a,4,5,6-hexahydro-8-cyclohexyl-1H-pyrazino-(3,2,1-j,k)-carbazolehydrochloride of the following formula:

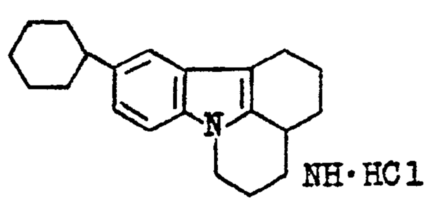

(I)

A method for preparing said novel compound, namely: 2,3,3a,4,5,6-hexahydro-8-cyclohexyl-1H-pyrazino(3,2,1-j,k)-carbazole hydrochloride of the formula:

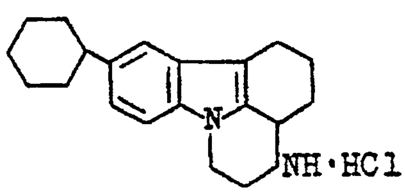

(I)

according to the present invention comprises alkylating 6-cyclohexyl-3,4-dihydrocarbazol-1(2H)-one with $\beta$-(N,N--dibenzylamino)ethylchloride hydrochloride in a two-phase system: an aromatic hydrocarbon - a 30-40% aqueous solution of an alkali in the presence of an interphase-transfer catalyst selected from the group consisting of tetraalkyl-, trialkylbenzyl, trialkyl(alkylbenzyl)ammonium halide, or a mixture of benzyl chloride and triethylamine, or dimethylformamide or polyethylene glycol, at a temperature within the range from 85 to 110°C, followed by hydrogenation of the resulting 6-cyclohexyl-9 $\beta$-(N,N-dibenzylamino)-ethyl-3,4-dihydrocarbazol-1 (2H)-one over a supported palladium catalyst in a medium of a lower aliphatic alcohol at a temperature from 50 to 70°C under a pressure of from 1 to 30 atm, followed by conversion of the resulting 2,3,3a,4,5,6-hexahydro-8-cyclohexyl-1H-pyrazino(3,2,1-j,k)-carbazole into its hydrochloride and isolation of the latter.

It is advisable to use, as the palladium catalyst, palladium bisacetylacetonate or palladium hydroxide and carbon of asbestos as a support. It is preferable to use as a calatyst, palladium bisacetylacetonate supported on carbon and to carry out the process at a temperature from 50 to 60°C under a pressure of 10 to 20 atm.

The compound according to the present invention exhibits an antidepressive activity. An antidepressive drug according to the present invention consisting of an active principle and a pharmaceutically acceptable vehicle incorporates, as the active principle, the compound according to the present invention, viz. 2, 3, 3a, 4, 6-hexahydro-8-cyclohexyl-1H-pyrazino(3,2,1-j,k)carbazole hydrochloride of the formula given hereinbefore.

The drug according to the present invention can be administered orally in any suitable form. It is preferred to use the drug according to the present invention in a tablet form. The drug according to the present invention in a tablet form should preferably contain the active principle in the amount of 25 mg per tablet.

The compound according to the present invention and a drug based thereon feature a pronounced antidepressive activity and is characterized by a number of characteristics inherent in antidepressants (lowers the depressive affect of reserpine, enhances the stimulant effect of d,l-amphetamine, l-dofa, 5-hydroxytryptophane and other pharmacological effects). As regards certain pharmacological parameters, the compound according to the present invention is 2-10 times more active than the known preparation pyrazidol.

The compound according to the present invention, like pyrazidol, exerts an inhibiting effect on the activity of monoaminoxidase, being a reversible selective inhibitor of monoaminoxidase of the A type.

Pharmacokinetically, the drug according to the present invention is characterized by a rapid resorptivity from the gastro-intestinal tract, a rapid penetration into the system blood flow and into the organs. The drug according to the present invention is characterized by a good penetration into the brain tissues and, in contrast to pyrazidol, it remains there for 3 to 6 hours as compared to at most 1.5 hour in the case of pyrazidol. The therapeutic effect comes on the 2nd - 5th day since the beginning of the treatment.

The drug according to the present invention is low toxic. The $LD_{50}$ when administered to white mice is 1.3 g/kg for rats it is 2.5 g/kg.

The drug according to the present invention provides no harmful effect on parenchymatous organs. It does not display any cardiotoxic effect, or carcinogenic, mutagenic, allergenic effects. The drug of the present invention does not affect the immune system of the body. It does not provide a cholinolytic effect. It is well tolerated by patients.

Best Mode for Carrying Out the Invention

The drug according to the present invention is a white crystalline powder with a yellowish shade to a light-yellow colour (green shade is permissible), very sparingly soluble in a 95% ethanol and chloroform, substantially insoluble in water and ether; m.p. 249-251°C (decomposition) (from methanol).

The structure of the compound was tested with elemental analysis, UV, NMR spectroscoly. It was also confirmed by mass-spectrography.

The compound according to the present invention and the drug based thereon were experimentally tested on animals and in clinics on human beings.

The compound according to the present invention was tested for a specific (antidepressive) activity.

The effect of the compound according to the present invention on the duration of immobilization in Porsolt's swimming behaviour test (Porsolt et al., 1977) in experiments on white mice of both sexes with a mass of 16-18 g. The compound according to the present invention in the doses of 1.0, 2.5 and 5 mg/l pyrazidol and imipramine in the doses of 10 and 25 mg/kg were administered to mice orally in a single dose and repeatedly during 5 and 18 days. Sixty minutes after the last (or a single) administration of the

preparations, the mice were placed into cylinders (10 cm diameter, 25 cm height) filled with water to 6 cm. The temperature of water was 21-22°C. The animals were observed for 5 minutes (since the 1st to the 6th minute) after placing them into the cylinders. The number and duration of all stops in swimming (immobilization) over the observation period were recorded.

The results are given in Table 1.

The compound according to the present invention noticeably reduced the time of immobilization of mice in swimming. At a single administration of the drug according to the present invention the effect was noted at the doses of the drug of 2.5 and 5 mg/kg, and at a repeated administration during 5 and 18 days the compound according to the present invention reduced the time of immobilization also in the doses of 1 mg/kg.

Pyrazidol at a single administration produced the effect only in the dose of 25 mg/kg, and at a repeated administration also in the dose of 10 mg/kg. Imipramine at a single administration was effective both in the dose of 10 mg/kg and 25 mg/kg. Upon a repeated administration the activity of the compounds was greater, as compared to the single administration, but no essential difference in activity was observed between the 5-days' and 18-days' administration.

Therefore, in Persolt's emotion-stress test the compound according to the present invention revealed a clearly pronounced protective effect, lowering depression of the animal and related immobilization. In this test it was active in doses 10 times lower than those of imipramine and pyrazidol.

Table 1

Effect of the Compound According to the Present Invention, Pyrazidol and Imipramine on the Duration of Immobilization in the Swimming Test

| Drugs | Doses, mg/kg orally | Duration of immobilization, seconds | | |
|---|---|---|---|---|
| | | Single administration | | |
| | | number of mice | Seconds, M±m | % |
| 1 | 2 | 3 | 4 | 5 |
| Distilled water | - | 80 | 158±8.6 | 100 |
| Compound of the present invention | 1.0 | 30 | 145±10.4 | 92 |
| | 2.5 | 30 | 111±9.9*** | 69 |
| | 5.0 | 40 | 105±8.1** | 63 |

Table 1 (continued)

| 1 | 2 | 3 | 4 | 5 |
|---|---|---|---|---|
| Distilled water | - | 40 | 176±6.5 | 100 |
| Pyrazidol | 10.0 | 40 | 164±5.9 | 93 |
| | 25.0 | 40 | 144±9.9*** | 82 |
| Distilled water | - | 30 | 153±7.2 | 100 |
| Imipramine | 10.0 | 30 | 131±5.6** | 88 |
| | 25.0 | 30 | 107±6.8* | 70 |

Table 1 (continued)

| Drugs | Duration of immobilization, seconds | | | | | |
|---|---|---|---|---|---|---|
| | 5-days' administration | | | 18-days' administration | | |
| | Number of mice | Seconds M±m | % | Number of mice | Seconds, M±m | % |
| 1 | 6 | 7 | 8 | 9 | 10 | 11 |
| Distilled water | 30 | 166±9.9 | 100 | 20 | 184±7.8 | 100 |
| Compound of the present invention | 30 | 102±8.1* | 61 | 20 | 107±8.0* | 58 |
| | 30 | 102±6.1* | 61 | 20 | 96±11.0* | 52 |
| | 30 | 88±5.2* | 53 | 20 | 75±9.8* | 41 |
| Distilled water | 30 | 163±7.2 | 100 | 20 | 184±7.8 | 100 |
| Pyrazidol | 30 | 142±5.7*** | 87 | 20 | 129±8.0* | 70 |
| | 30 | 116±8.1* | 71 | 20 | 112±8.6* | 61 |
| Distilled water | 20 | 157±5.9 | 100 | 20 | 184±7.8 | 100 |
| Imipramine | 30 | 92±5.9* | 59 | 20 | 96±9.8* | 52 |
| | 30 | 61±6.3* | 39 | 20 | 66±9.8* | 36 |

Difference from the control statistically significant:
*) $p < 0.001$;
**) $p < 0.02$;
***) $p < 0.05$.

The compound according to the present invention was tested for the ability to decrease depriming effects of reserpin (blepharoptosis, hypothermia and the like).

Tests were carried out on white mice of both sexes with a mass of 16-18 g.

The compound according to the present invention was orally administered in a single dose and repeatedly during 18 days.

The compound according to the present invention was administered in the single doses of 1, 2.5 and 5 mg/kg; repeatedly - in the doses of 0.5, 1.0, 2.5 and 5 mg/kg. The single-time administration and the last administration of the 18 days' course were effected 60 minutes prior to the intraperitoneal administration of reserpine (2.5 mg/kg). For a comparative assessment in these and other experiments pyrazidol and imipramine were also administered. The magnitude of blepharoptosis was assessed agninst a 4-points' scale by Rubin et al. (1957) 4 hours after administration of reserpine, since during this period the depriming effects of reserpine were revealed to the fullest extent (according to the data by Chen. et al., 1954).

The results of this study are shown in Table 2 hereinbelow.

The results of the tests showed that the compound according to the present invention, as regards its anti-reserpine effect, was superior to pyrazidol in acute and chronical experiments by more than 10 times; it was also more active than imipramine.

Table 2

Effect of the Compound According to the Present Invention, Pyrazidol and Imipramine on Reserpine-Induced Blepharoptosis in Mice

| Drugs | Doses, mg/kg orally | Effect 4 hours after administration of reserpin (2.5 mg/kg, intraperitoneally) | | | | | |
|---|---|---|---|---|---|---|---|
| | | Single administration | | | 18-days' administration | | |
| | | Number of mice | Blepharoptosis, points (M±m) | % | Number of mice | Blepharoptosis, points (M±m) | % |
| 1 | 2 | 3 | 4 | 5 | 6 | 7 | 8 |
| Distilled water | - | 60 | 3.2±0.18 | 100 | 30 | 3.5±0.09 | 100 |
| Compound of the invention | 0.5 | | | | 10 | 2.6±0.21* | 65 |
| Ditto | 1.0 | 24 | 2.2±0.18* | 66 | 10 | 1.1±0.32* | 31 |
| Ditto | 2.5 | 30 | 0.6±0.18* | 17 | 30 | 0.4±0.09* | 10 |
| Ditto | 5.0 | 24 | 0.3±0.18* | 9 | 10 | 0.1±0.21* | 2,5 |
| Pyrazidol | 10.0 | 84 | 2.3±0.14* | 69 | 6 | 0.7±0.35* | 20 |
| Ditto | 25.0 | 72 | 1.7±0.18* | 51 | 12 | 0.5±0.18* | 14 |
| Imipramine | 10.0 | 66 | 1.9±0.14* | 58 | 12 | 0.7±0.18* | 20 |
| Ditto | 25.0 | 60 | 1.2±0.14* | 36 | 6 | 0.5±0.35* | 14 |

*) The difference from the control was statistically significant at $P < 0.001$.

The compound according to the present invention was also tested for its influence on the effects of d,l-amphetamine (stereotypy, hyperlocomotion and the like).

To evaluate the influence on stereotypy, the experiments were carried out on white rats of both sexes with a mass of 100-150 g. The compound according to the present invention, pyrazidol and imipramine were administered orally 60 minutes before a hypodermal administration of d,l-amphetamine (5 mg/kg). Stereotypy was assessed in points of from to 3 (after Janssen et al., 1967).

The results of the tests are shown in Table 3 hereinbelow.

The experiments showed that the compound according to the present invention enhanced stereotypy and was close to pyrazidol and imipramine in respect of this parameter.

The influence on hyperlocomotion induced by d,l-amphetamine was studied in experiments on male white mice with a mass of 20-22 g.

The compound according to the present invention, pyrazidol and imipramine were administered orally 60 minutes before an intraperitoneal administration of d,l-amphetamine in the dose of 2.5 mg/kg. The locomotive activity of the mice was recorded 10-20 minutes after administration of d,l-amphetamine for 10 minutes.

The results of the tests are shown in Table 4 hereinbelow.

The compound according to the present invention in the doses 1.0, 2.5 and 5 mg/kg enhanced the hyperlocomotive effect of d,l -amphetamine.

Pyrazidol and imipramine under similar experimental conditions did not cause an increase in the effect of d,l-amphetamine. The enhanced effects of stereotypy and hyperlocomotion induced by d;l-amphetamine could be attributed to enhanced adrenergic and dophaminergic processes in the brain.

Table 3

Enhanced Effect of the Compound According to the Present Invention on Stereotypy Induced in Rats by d,l-Amphetamine

| Drugs | Oral dose, mg/kg | Number of rats | Stereotypy (tolal, for 4 measurements for 4 hours after hypodermal administration of d,l-amphetamine) | |
|---|---|---|---|---|
| | | | Points | % |
| 1 | 2 | 3 | 4 | 5 |
| Destilled water | - | 60 | 2.8±0.14 | 100 |
| Compound of the invention | 2.5 | 10 | 4.0±0.28* | 145 |
| Ditto | 5.0 | 30 | 4.8±0.14* | 170 |
| Ditto | 10.0 | 40 | 6.3±0.14* | 225 |
| Pyrazidol | 5.0 | 50 | 4.6±0.14* | 165 |
| Ditto | 10.0 | 45 | 5.9±0.14* | 210 |
| Imipramine | 5.0 | 30 | 5.6±0.14* | 200 |
| Ditto | 10.0 | 15 | 8.6±0.23* | 306 |

*) The difference from the control was statistically significant at $P < 0.001$.

The compound according to the present invention was tasted for the exciting effect of 5-hydroxytryptophane (head shaking).

The experiments were conducted on white mice of both sexes with a mass of from 16 to 20 g following the procedure suggested by Corne et al. (1963).

Table 4

Effect of the Compound According to the Present Invention, Pyrazidol and Imipramine on Hyperlocomotion in Mice Induced by d,l-Amphetamine

| Drugs | Doses, oral, mg/kg | Number of mice groups (5 mice in each) | Average value of locomotor activity of a group of 5 mice for 10 minutes groups 10-20 minutes after administration (5 mice of d,l-amphetamine (2.5 mg/kg intra in each) peritoneally) |
|---|---|---|---|
| 1 | 2 | 3 | 4 |
| Distilled water | - | 20 | 1,924.5±177.6 |
| Compound of the invention | 1.0 | 12 | 3,482.6±445.5* |
| Ditto | 2.5 | 10 | 3,315.5±462.6** |
| Ditto | 5.0 | 10 | 4,885.0±537.2** |
| Pyrazidol | 5.0 | 10 | 1,440.0±380.2 |
| Ditto | 10.0 | 10 | 1,926.0±119.9 |
| Imipramine | 5.0 | 12 | 1,440.3±365.2 |
| Ditto | 10.0 | 12 | 2,024.5±221.7 |

The difference from the control was statistically significant at:

*) $P < 0.01$;

**) $P < 0.02$.

The compound according to the present invention in the doses of 1.0, 2.5, 5.0 and 10 mg/kg was

administered orally 60 minutes prior to an intraperitoneal administration of 5-hydroxytryptophane (50 mg/kg). In this dose 5-hydroxytryptophane did not cause head shaking in mice, but the development of this phenomenon in some animals was noted under the influence of the compound according to the present invention and pyrazidol.

To asses this effect, the number of mice in which at least one shaking of the head was observed for 2 minutes 25 minutes after administration of 5-hydroxytryptophane was recorded and the $ED_{50}$, i.e. the dose at which bead shaking were noticed in 50% of the animals, was calculated.

In these experiments the $ED_{50}$ for the compound according to the present invention was 2.5 (2.1-3.0) mg/kg (orally); that of pyrazidol - 20 (14-28) mg/kg (orally).

Therefore, regarding the ability of enhancing the convulsiv activity of 5-hydroxytryptophane in mice the compound according to the present invention was considerably more active the pyrazidol (by about 8 times) which could be assigned to a much higher, than in the case of pyrazidol, serotoninergic effect of the compound according to the present invention.

The ability of the compound of this invention to enhance the convulsive activity of 5-hydroxytryptophane enabled assumption of the presence of an inhibiting effect on monoaminoxidase therein.

The effect of the compound according to the present invention on the activity of monoaminoxidase was studied in special biochemical investigations.

The experiments were conducted in vivo on male white rats with a mass of 180-200 g. The compound in the doses of 5, 10, 25 and 50 mg/kg was administered orally. An equal volume of a 0.97 solution of NaCl was administered to control animals. 1.5 hour after administration of the drug the animals were killed. In study of the duration of the effect of the compound according to the present invention the animals were killed within different time limits (1.5, and 48 hours) after administration of the ccmpound orally in the dose of 25 mg/kg. The activity of monoaminoxidase was determined in 50% homogenates of the liver and brains of rate prepared on the basis of a 0.1 M phosphate buffer (pH = 7.4) containing 2% of a detergent - Trytol X-100. The substrates were used in concentrations of serotonin 10 µmol, tyramine 6 µmol per sample. The activity of monoaminoxidase was evaluated by liberation of ammonia during incubation of the samples for 50 minutes at the temperature of 37°C in an oxygen atmosphere.

Protein in homogenates of the rat liver and brain was determined following the Peterson procedure (1977). The results of the experiments showed that the compound according to the present invention produced a pronounced antimonoaminoxidase effect superior to that of pyrazidol.

The monoaminoxidase-inhibiting effect shown by the compound according to the present invention, likewise the effect produced by pyrazidol, features - substrate und tissue selectivity: the compound according to the present invention inhibits, to a greater extent, the oxidizing desamination of serotonin than of tyramine and suppresses activity of menoaminoxidase of the brain to a greater extent than that of the liver. The antimonoaminoxidase effect of the compound according to the present invention is noted already in the dose of 5 mg/kg and increases with the growth of doses so that substrate selectivity of its effect is fully retained in the liver and decreases in the brain. The obtained results are shown in Table 5 hereinbelow.

As it is seen from the Table, the compound according to the present invention provides a selective inhibiting monoaminoxidase effect in a small dose of 5 mg/kg which is more pronounced in the brain than in the liver. The compound according to the present invention noticeably inhibits deamination of serotonin (by 60%) and considerably lesser - that of tyramine in the brain. Upon elevation of its dose up to 50 mg/kg its inhibiting effect on monoaminoxidase of the A type increases.

With the dose of the drug of 5 mg/kg and 50 mg/kg a considerable difference is retained as regards the effect on deamination of sorotonin and tyramine. The compound according to the present invention in all the doses employed does not affect deamination of tyramine in the liver.

The study of the duration of the antimonoaminoxidase effect exerted by the compound according to the present invention showed that in the dose of 25 mg/kg became pronounced to the maximum extent within 1.5 hour, while after 6 hours the drug inhibites mainly deamination of serotonin in the brain and liver, and that of tyramine in the brain. After 48 hours the activity of monoaminoxidase was fully restored. The results of the investigation are shown in Table 6 hereinbelow. After administration of pyrazidol the activity of monoaminoxidase of the brain was fully restored after 24 hours, that of the liver - after 6 hours.

Therefore, the antimonoaminoxidase effect of the compound according to the present invention, likewise that of pyrazidol, is short and reversible and relate mainly to monoaminoxidase of the A type.

Table 5

Effect of the Compound According to the Present Invention on Oxidizing Deamination of Monoamines in Homogenates of the Rats' Brain and Liver

| Drug | Doses, oral, mg/kg | Inhibition of deamination, % (M±m) | | | |
|---|---|---|---|---|---|
| | | Brain | | Liver | |
| | | Serotonin | Tyramine | Serotonin | Tyramine |
| 1 | 2 | 3 | 4 | 5 | 6 |
| Compound of the invention | 5 | 60±6.3 | 17±4.2 | 36±9.0 | 0 |
| Ditto | 10 | 61±7.6 | 16.6±3.4 | 27±6.7 | 0 |
| Ditto | 25 | 71±7.6 | 40±3.0 | 64±6.4 | 0 |
| Ditto | 50 | 93±3.0 | 43±6.6 | 89±2.7 | 0 |

Table 6

Duration of Inhibition of the Activity of Monoaminoxidase in Rats' Tissues of Liver and Brain by the Compound According to the Present Invention (25 mg/kg orally)

| Time, hours | Inhibition of deamination, % (M±m) | | | |
|---|---|---|---|---|
| | Brain | | Liver | |
| | Serotonin | Tyramine | Serotonin | Tyramine |
| 1 | 2 | 3 | 4 | 5 |
| 1.5 | 71.0±2.0 | 40.0±3.0 | 64.0±6.4 | 0 |
| 6.0 | 66.5±3.0 | 36.0±9.3 | 53.0±3.0 | 0 |
| 48.0 | 14.0±4.8 | 2.5±1.1 | 0 | 0 |

The study of the effect on the bioelectrical activity of the brain was effected in acute experiments using 8 consious cats immobilized with diplacin with a mass of 2.5-3.4 kg under conditions of a controlled breathing and 25 rabbits with a mass of 2.6-3.1 kg with chronically implanted electrodes. Recorded on an electroencephalograph was an electroencephalogram of the sensomotor, parietal and visual regions of the cerebral cortex in cats, plus that of the reticular formation of the midbrain, dorsal hippocamp and amygdala in rabbits.

The compound according to the present invention in the doses of 0.5 and 1 mg/kg, when administered

intravenously and intraperitoneally did not bring about any changes in the bioelectric activity in the cortex and subcortical brain structures. In the case of the dose of 2 mg/kg a progressive increase in frequency of the rhythm of oscillations of biopotentials to 20-25 per second was observed in cats, along with reduction of their amplitudes to 25-40 μV (in the control the frequency of oscillations was 8-10 per second, the amplitude - 90-120 μV) in all of the recorded regions of the brain cortex. In rabbits a low-amplitude high-frequency rhythm dominated in the sensomotor region of the cerebral cortex in rabbits after administration of the drug according to the present invention, while in the visual, parietal and reticular formation of the midbrain regular low-amplitude rhythms with a frequency of 5-7 oscillations per second dominated. In the hippocampus a teta-activity was recorded: sinusoidal regular waves with a frequency of 4-6 oscillations per second and with an amplitude of 150-200 μV. These variations in the electroencephalogram appeared 75-90 minutes after administration of the drug.

In the dose of 5 mg/kg the compound according to the present invention caused changes in the elektroencephalogram characteristic for the cose of 2 mg/kg, but the effect of activation was recorded faster, i.e. within 45-60 minutes after administration. The activation of the elektroencephalogram caused by the drug was intermittently interrupted by periods of oscillations of different amplitudes and frequences characteristic for the control background. No continuous activation was observed, as a rule.

Pyrazidol in doses from 1 to 5 mg/kg (intravenously) also produced a predominantly activating effect on the electroencephalogram of the cortex and subcortical structures of the brain.

Tricyclic antidepressants, namely: imipramine and amitryptyline, in contrast to the compound according to the present invention and pyrazidol, produced a synchronizing effect and caused domination of a slow high-amplitude activity on the electroencephalogram.

Also studied was the effect of the compound according to the present invention on reproduction of the memory traces upon amnesia.

The experiments were carried out using male rats of a mass of 180-220 g.

The antiamnestic effect of the compound according to the present invention was assessed by the method of a conditional reflex of a passive avoidance with a subsequent use, as amnesic factors, of an electric shock (50 mA, 0.3 ms) through ear electrodes or scopolamine (1 mg/kg hypodermally).

The learning of the conditional reflex of a passive avoidance was effected by the "Step-down" method (Izquerdo, 1981) in an experimental unit consisting of a 30x30x40 cm chamber with an electrified floor having in its centre a plastic platform of 10x15x0.5 cm size. A rat was placed onto the platform and a latent period of its escape therefrom was recorded (in seconds). When escaping the platform onto the floor, the rat was subjected to an electric irritation on paws (0.5 mA, 10 seconds) that made it to climb the platform. The learning course took 6 sessions, each of 3 minutes duration. The conditional response was regarded as acquired if an animal remained on the platform for 1 minute.

The amnesic effect was produced immediately after learning course and 30 minutes thereafter the compound according to the present invention was administered in the doses of 1 and 3 mg/kg or (for comparison) pyracetam was administered in the doses of 100, 300, 600 and 1,000 mg/kg. The reproduction of the memory trace was assessed 24 hours after the electric shock and 2.5 hours after administration of scopolamine.

The thus-obtained results are shown in Table 7 hereinbelow.

As it is seen from the Table 7, the compound according to the present invention in the dose of 3 mg/kg statistically significant increases the number with a restored habit of the conditional reflex of a passive avoidance disturbed under the influence of an electric shock and scopolamine. Pyracetam produced a similar effect only in the dose of 1,000 mg/kg.

The correction of the memory trace, disturbed by amnesia, under the influence of the compound according to the present invention can also demonstrate its accompamying nootropic effect.

Table 7

Effect of the Compound According to the Present Invention and Pyracetam on Restoration of the Conditional Response of Passive Avoidance (antiamnestic effect)

| Drugs | Doses, oral, mg/kg | Number of rats | Restoration of the disturbed conditional response of passive avoidance | |
|---|---|---|---|---|
| | | | Number of rats, %, remained on the platform for 1 minute | |
| | | | 1 day after electric shock | 2.5 hours after administration of scopolamine (1 mg/kg hypodermally) |
| 1 | 2 | 3 | 4 | 5 |
| Destilled water | - | 20 | 65±2.0 | 65±2.0 |
| Electroshock | - | 20 | 14.2±3.0 | 10±3.3 |
| Compound of the invention + electroshock | 1 | 20 | 25±3.2 | 22±6.0 |
| Compound of the invention + electroshock | 3 | 20 | 40±6.0* | 33±4.4* |
| Pyracetam + electroshock | 100 | 10 | 12±3.3 | 12±5.5 |
| Ditto | 300 | 10 | 13±4.2 | 13±1.6 |
| Ditto | 600 | 10 | 12±3.3 | 20±5.5 |
| Ditto | 1000 | 10 | 46±6.4 | 52±2.0* |

The difference from the control was statistically significant at *) $P < 0.001$

The compound according to the present invention was alsc tested for its effect on the cardiovascular system. The compound according to the present invention in doses of 1-100 mg/kg orally produced no essential effect on the arterial pressure and heart beat rate in normotensive rats of the Vistar line in the case of a free behaviour (the arterial pressure measurements were effected in the tail area prior to administration of the drug and 1.3 and 6 hours thereafter). A daily administration, for 5 days, of the

compound according to the present invention to normotensive rats in a free behaviour in the oral dose of 10 mg/kg did not change the heart beat rate on the first or the following days.

The determination of the influence produced by antidepressants-inhibitors of monoxidase on the pressor effect of tyramine has a great importance in the assessment of their safety.

The experiments were conducted on conscious normotensive female rats of the Vistar line with a mass of 210-250 g placed under conditions of a free behaviour. Tyramine was orally administered in the dose of 20 mg/kg prior to (control) and 1.3, 6, 18 and 24 hours after administration of the compound according to the present invention and of the drug Moclobemide (antidepressant-inhibitor of monoaminoxidase of the A type of a reversible action) - for the sake of comparison. In these experiments it was found that in the dose of 25 mg/kg (orally) the compound according to the present invention, likewise Moclobemide in the dose of 25 mg/kg orally, enhanced the pressor effect of tyramine by 2 times. The enhanced effect was noticed 6-18 hours after administration of the compound according to the present invention.

Upon administration of the compound according to the present invention in the dose of 5 mg/kg (orally) for 5 days no enhanced effect of tyramine was observed. Moclobemide in the dose of 5 mg/kg (orally) even upon a single administration enhanced the pressor response to tyramine.

The compound according to the present invention was also tested for its central and peripheral anticholinergic effect. The compound of this invention in the doses of 10 and 25 mg/kg orally and 1.0, 2.0 and 5.0 mg/kg intravenously did not lower the convulsive activity of oxotremorine and its hypothermal effect: neither it lowered the intensity and duration of arecoline hyperkineses, as well as the activating effect of galantamine on the bioelectrical activity of the brain. These data attest to the absence of the anticholinergic effect in the compound according to the present invention.

The acute and chronical toxicity of the compound according to the present invention was studied on mice, rats and rabbits.

The results of these tests showed that the compound according to the present invention was low-toxic: the $LD_{50}$ upon oral administration of the drug to mice was 1,300 mg/kg to rats - 2,500 mg/kg.

Upon a daily administration of the compound according to the present invention for the period of 6 months no functional disturbances on the part of the inner organs of the animals were observed. The drug produced no essential influence on the state of the central nervous and cardiovascular systems, did not change the morphological and biochemical characteristics of blood.

A pathomorphological study revealed no pathological changes upon administration of the compound according to the present invention in the doses of 10 and 50 mg/kg which exceeded the purported therapeutic dose by 2 and 10 times respectively. The compound according to the present invention, when administered in the dose of 100 mg/kg (20 times higher than the therapeutic dose) caused non-specific pathological changes in parenchymatous organs which were of a reversible character. Upon investigation of the test animals' organs one month after discontinuation of administration of the compound according to the present invention no pathological changes were observed in the internal organs.

The compound according to the present invention was also tested for a possible mutagenous effect by methods of evaluation of genetic mutations on fruit fly: dominant lethal mutations were studied on embryonic cells of rats, chromosome aberrations - in bone-marrow cells of mice and in a culture of human lymphocytes.

The drug revealed no mutagenic properties in any of the performed tests.

The drug according to the present invention did not reveal carcinogenic properties when tested in a chronic experiment on mice and rats. The experiments showed that the compound according to the present invention did not exhibit an allergenic effect, an embryotoxic or teratogenic effect.

The drug according to the present invention was tested in a clinic on patients (561 persons) aged 18 to 72 suffering from depressions of various genesis - endogenous, reactive, neurotic depressions caused by organic injuries of the brain, as well as depressive disturbances in chronical alcoholism and the like.

Clinical tests for different types of depressions were carried out in the following groups of patients:

| | |
|---|---|
| maniac-depressive psychosis and cyclothymia | 164 |
| neurotic depression | 77 |
| schizophrenia | 252 |
| reactive depression | 11 |
| organic diseases of the central nervous system (of vascular, traumatic and infectional genesis) | 18 |
| involutional depression | 9 |
| chronic alcoholism | 26 |
| others | 4 |

The drug according to the present invention was administered orally in tablets twice a day - in the morning and in the afternoon. The dose depended on the severity of disease, efficiency and tolerance of the drug.

The treatment was usually started with administration of the drug in single doses of 0.025 g (25 mg) with a gradual increment of the daily dose for 1 to 3 weeks up to 200-300 to 350-400 mg.

The treatment duration ranged from 7 days to 12 weeks, more frequently 40-45 days.

In a number of cases (55 patients) the drug according to the present invention was administered in combination with other psychotropic agents selected mainly from the group of tranquilizers and neuroleptics (tazepam, seduxen, tizercin, and the like).

The efficiency as the drug according to the present invention was assessed with the use of assessment scales.

In the analysis of the effect produced by the compound according to the present invention it was found that one of its most important features was a rapid development of the effect: in the majority of patients already on the 2-nd - 3-rd days of the treatment the stimulating effect of the drug appeared which revealed first of all in a less pronounced asthenic symptomatology: weakness, flabbiness, increased fatiguability; a revived motility and facial expressions, as well as improvement of thinking were noticed. This effect of the drug according to the present invention developed faster than in the case of other antidepressants.

Against the background of the primary psychostimulant effect of the drug according to the present invention a proper thymoleptic effect started to get clearly pronounced on the 6-th - 10-th day of the treatment which was manifested in higher spirits, reduced depersonalization and derealization disturbances, normalization of sleep and appetite.

The analysis of the efficiency of the drug according to the present invention in various kinds of depression showed that this drug was especially effective in depressive disorders of the neurotic and reactive genesis. In the therapy of patients suffering from these depressions an improvement was observed in 80-100% of cases. In the case of endogenous depressions within the limits of maniac-depressive psychosis and schizophrenia the effect of the drug according to the present invention was noted mainly with predomination of asthenic or asthenoadynamic syndromes and, especially, in the states of a light and medium severity. In such cases an improvement was observed in 37-92% of the events (50% on the average).

The drug according to the present invention was highly effective in patients (6 out of 21) with a previous resistance to another antidepressant therapy.

In patients with symptomatic alcoholism against the background of psychic disorders the preparation according to the present invention individually or in combination with a conventional therapy caused an improvement of mood sleep, somatovegetative disturbances already on the 3-rd day, lowering the urge for alcohol in the case of an alcohol withdrawl syndrome. In the treatment with the drug according to the present invention the rest patients more easily tolerated the phenomena of abstinence than the patients who were not administered with the preparation.

The drug according to the present invention manifested a good toleration by patients. Side effects (sleep disorders, somnolence, a more pronounced feeling of anxiety, dizziness, allergic responses) appeared in some cases during the first days of the treatment course and frequently disappeared spontaneously or upon lowering of the dose of the drug.

The study of the somatovegetative sphere revealed no essential change as compared to the background characteristics, in arterial pressure, body temperature, clinical and biochemical analyses of blood and urine, disorders in the gastrointestinal tract, and the like.

Therefore, the drug according to the present invention is, a novel effective antidepressant with a predominantly stimulating component of its action. An important feature of its action is a rapid occurrence of the therapeutic effect.

No cholinolytic properties were found the drug according to the present invention and, in contrast to the antidepressants of this series, it provided no cardiotoxic effect. This is especially important in the treatment of depressive states in persons with a poor tolerance in respect of other antidepressants and especially in aged people. The drug according to the present invention is indicated for administration in the case of nosologically different depressive states (endogenous, reactive, organic depressions), mainly in light depressions and depressive disturbances of a medium severity with astenic-adynamic symptoms, as well as in therapy of so-called classic depressions.

The drug according to the present invention is usually well tolerated by patients of different age, wherefore it can be administered to both clinical and ambulatory patients, aged and weakened patients.

The drug according to the present invention can be administered in the form of tablets, capsules, pellets. A preferable pharmaceutical form is tablets. A single dose is 25-100 mg, a daily dose - 150-400 mg. The duration of the treatment course is 2 to 6 weeks. The total course dose is 0.75 to 16.8 g. The effect of the drug according to the present invention is developed on the 2nd - 5th day of the treatment.

The present invention also relates to a method for preparing the compound according to the invention, viz. 2,3, 3a,4,5,6-hexahydro-8-cyclohexyl-1H-pyrazino(3,2,1-j,k)carbazole hydrochloride (formula I).

The method for preparing the compound according to the present invention is carried out according to the following sheme:

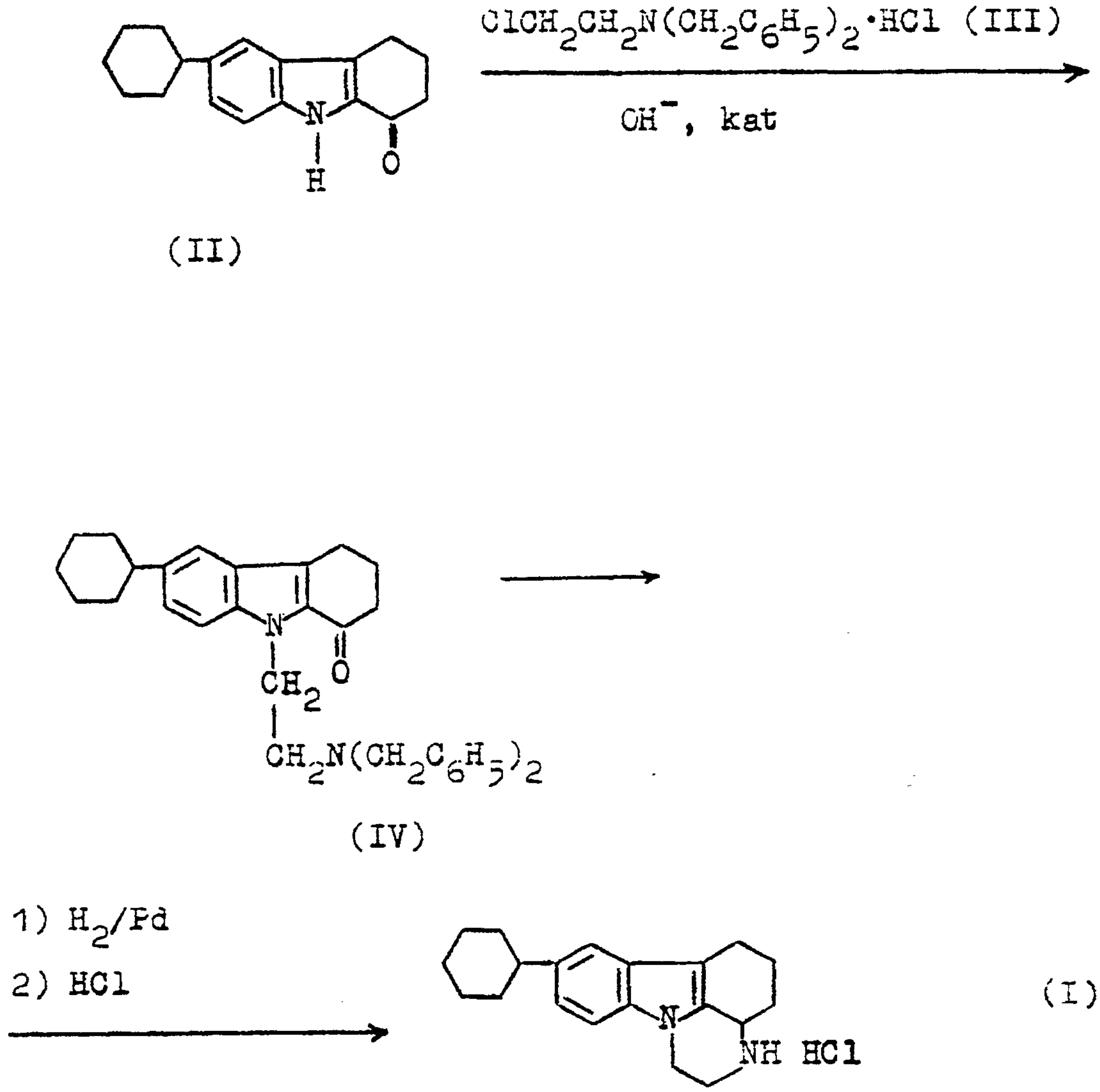


(II)

(IV)

(I)

6-Cyclohexyl-3,4-dihydrocarbazol-1(2H)-one (II) is subjected to alkylation with $\beta$ -(N,N-dibenzylamino)-ethylchloride hydrochloride (III) by the method of the interphase catalysis in a two-phase system: an aromatic hydrocarbon (e.g. chlorobenzene, toluene) and a 30-40% aqueous solution of an alkali at a temperature of 85-110°C in the presence of an interphase transfer catalyst.

As the interphase-transfer catalyst a tetra-alkyl- or a trialkylbenzyl, or a trialkyl(alkylbenzyl)-ammonium halide can be used such as, for example, tetrabutylammonium bromide, triethylbenzylammonium chloride. A mixture of benzyl chloride und triethylamine, or dimethylformamide, or polyethylene glycol can be used. The resulting 6-cyclohexyl-9--$\beta$-(N,N-dibenzylamino)ethyl-3,4-dihydrocarbazol-1(2H)one (IV) is hydrogenated over a palladium catalyst supported on a carrier such as carbon or a non-combustible carrier, e.g. asbestos. The process is carried out in a medium of a lower alcohol at a temperature ranging from 50 to 70°Cunder a pressure from 1 to 30 atm. It is preferable to use as a catalyst, palladium bisacetylacetonate or palladium hydroxide on carbon or asbestos. In the case of using palladium bisacetylacetonate supported on carbon the process should be preferably carried out at a temperature of 50-60°C under a pressure of 10 to 20 atm. The thus prepared 2,3,3a,4,5,6-hexahydro-8-cyclohexyl-1H-pyrazino (3,2,1-j,k)carbazole is converted into hydrochloride and the desired product is isolated. The yield of the desired product is equal to 70-75% by mass as calculated for the starting compound (II).

For a better understanding of the present invention, sore specific examples illustrating the process for the preparation of the compound according to the present invention are given hereinbelow.

Example 1

To 100 ml of toluene and 100 ml of a 30% aqusous solution of caustic soda 20 g of a 98% (0.0733

g/mol) 6-cyclohexyl-3,4-dihydrocarbazol-1(2H)-one (compound II) were added and, after stirring for 5-10 minutes, 27.75 g of a 96% (0.0899 g/mol) of β -(N,N-dibenzylamino)ethylchloride hydrochloride and 0.4 g of tetrabutylammonium chloride were added thereto. The reaction mixture was stirred and heated for 5-6 hours at a temperature of 95-100°C. On completion of the reaction (control by the method of thin-layer chromatography), the layers were separated, the aqueous layer was extracted with toluene, the toluene extract was combined with the main toluene layer. Toluene was distilled-off in vacuum, the residue was dissolved upon heating in 100 ml of absolute ethanol. The crystalline residue precipitated after cooling was filtered-off, washed with 15 ml of absolute ethanol and dried.

There were thus obtained 34.0 g (94.52% by mass) of substantially pure 6-cyclohexyl-9-β -(N,N-dibenzylamino)-ethyl-3,4-dihydrocarbazol-1(2H)-one in the form of a light-yellow crystalline substance melting at 103-104°C(m.p. 104-105°C from ethanol).

Found, %: C 83.56, H 7.90, N 5.64 $C_{34}H_{38}N_2O$

Calculated, %: C 83.22, H 7.8, N 5.71.

IR-spectrum, $\nu_{CO}$ 1,650 $cm^{-1}$,

UV-spectrum (ethanol): λ , nm (lg ε ): 241.9 (4.27), 314.7 (4.34).

Thereafter, a suspension of 10 g (0.0204 g/mol) of 6-cyclohexyl-9 β-(N,N-dibenzylamino)ethyl-3,4-dihydrocarbazol-1(2H)-one in 60 ml of methanol was hydrogenated with hydrogen in the presence of 2 g of a 5% palladium hydroxide supported on carbon under the pressure of 10 atm and at the temperature of 60°C till hydrogen absorption was stopped. Usually, a theoretically calculated amount of hydrogen was absorbed. On completion of the reaction the catalyst was filtered-off and the filtrate was treated with a concentrated hydrochloric acid to a pH of 1-2 (3-4 ml). The resulting residue was filtered-off after cooling, washed with acetone and dried.

There were thus obtained 5.75 g (80.66% by mass, as calculated for the starting compound II) of commercial 2,3,3a,4,5,6-hexahydro-8-cyclohexyl-1H-pyrazino(3,2,1-j,k)carbazole hydrochloride in the form of a crystalline substance of a white colour with a slightly yellowish shade. After recrystallization (from aqueous methanol) 5.25 g (73.6% by mass, as calculated for the starting compound II) of 2,3,3a,4,5,6-hexahydro-8-cyclohexyl-1H-pyrazino(3,2,1-j,k)carbazole hydrochloride were obtained.

UV-spectrum, $\nu_{max}$, nm, (lg ε): 229,5 (4.651), 276.7 (3.132).

Mass-spectrum, m/z: $M^+$ 330.9.

NMR¹H-spectrum (chemical shifts, ppm): H-1 4.15, H-2 4.38; H-4 3.12, H-5 3.02, H-6 2.29, H-7 7.58, H-9 7.49, H-10 7.49; H-1' 2.62, H-2', H-3', H-4' 1.2-1.9 ppm.

Example 2

To 100 ml of toluene and 100 ml of a 30% solution of caustic soda 20 g of a 98% 6-cyclohexyl-3,4-dihydrocarbazol-1(2H)-one (0.0733 g/mol) were added and, after stirring for 5-10 minutes, 27.75 g of a 96% β-(N,N-dibenzylamino)ethylchloride hydrochloride (0.0899 g-mol) and 0.4 g of triethylbenzylammonium chloride were added thereto. The reaction was conducted in a manner similar to that described in the foregoing Example 1 to give 34.35 g (95.5% by mass) of 6-cyclohexyl-9β-(N,N-dibenzylamino)-ethyl-3,4-dihydrocarbazol-1(2H)-one melting at 103-104°C.

A suspension of 10 g (0.0204 g/mol) of 6-cyclohexyl--9 β -(N,N-dibenzylamino)ethyl-3,4-dihydrocarbazol-1(2H)-onein 60 ml of methanol was hydrogenated with hydrogen in the presence of 0.35 g of palladium bisacetylacetonate and 2.5 g of activated carbon under a pressure of 20 atm and at a temperature of 50°C. On completion of the reaction, the catalyst was filtered-off and the filtrate was treated with a concentrated hydrochloric acid to a pH of 1-2. The precipitate formed after cooling was filtered-off, washed with acetone and dried to give 5.87 g (83.17% by mass, as calculated for the starting compound II) of a commercial desired product. After recrystallization (from aqueous methanol) 5.12 g (72.54% by mass as calculated for the starting compound II) of 2,3,3a,4,5, 6-hexahydro-8-cyclohexyl-1H-pyrazino(3,2,1-j,k)-carbazole hydrochloride were obtained. The characteristics of the compound were similar to those specified in Example 1 hereinabove.

Example 3

To 100 ml of chlorobenzene and 100 ml of a 40% solution of sodium chloride 20g of a 98% (0.0735 g/mol) 6-cyclohexyl-3,4-dihydrocarbazol-1H(2H)-one and after stirring for 5-10 minutes 27.75 g (0.0899 g/ml) of a 96% β-(N,N-dibenzylamino)ethylchloride hydrochloride and 0.5 ml of benzyl chloride along with 0.6 ml of triethylamine were added thereto. The reaction was conducted in a manner similar to that described in Example 1 hereinbefore, at the boiling temperature of the reaction mass.

In this manner 34.73 g (96.55% by mass) of 6-cyclohexyl-9 β-(N,N-dibenzylamino)ethyl-3,4-dihydrocarbazol-1(2H)-one melting of 103-104°C were obtained.

A suspension of 10 g (0.0204 g/mol) of 6-cyclohexyl-9 β-(N,N-dibenzylamino)ethyl-3,4-dihydrocarbazol-1(2H)-one in 60 ml of methanol was hydrogenated with hydrogen in the presence of 0.35 g of palladium bisacetylacetonate and 2.5 g of activated carbon under the pressure of 1-3 atm and at the temperature of 60°C.

On completion of the reaction, the catalyst was filtered-off,the filtrate was added with 60 ml of acetone and treated with a concentrated hydrochloric acid to a pH of 1-2. The precipitate thus farmed was filtered-off after cooling, washed with acetone and dried.

There were thus obtained 5.75 g (82.37% by mass as calculated for the starting compound II) of a commercial) desired product. After recrystallization (from aqueous methanol) 5.20 g (74.49% by mass as calculated for the starting compound II) of 2,3,3a,4,5,6-hexahydro8-cyclohexyl-1H-pyrazino(3,2,1-j,k)-carbazole hydrochloride were obtained. The thus-prepared compound had the characteristics similar to those described in Example 1 hereinbefore.

Example 4

To 100 ml of toluene and 100 ml of a 40% solution of caustic soda 20 g (0.0735 g/mol) of a 98% 6-cyclohexyl-3,4-dihydrocarbazol-1(2H)-one (II) were added and after stirring for 5-10 minutes 27.75 g (0.0899 g/mol) of a 96% β -(N,N-dibenzylamino)ethylchloride hydrochloride and 1 ml of benzyl chloride and 1.2 ml of dimethylformamide were added thereto. The reaction was conducted in a manner similar to that described in Example 1 hereinbefore.

There were thus obtained 34.21 g (95.1% by mass) of 6-cyclohexyl-9 β -(N,N-dibenzylamino)ethyl-3,4-dihydrocarbazol-1(2H)-one (IV) melting at 103-104°C.

Then the further steps of the process were carried out as described in Example 1 to give 5.76 g (81.27% by mass as calculated for the starting compound II) of a commercial desired product. After recrystallization (from aqueous methanol) 5.09 g (71.82% by mass as calculated for the starting compound II) of 2,3,3a,4,5,6-hexahydro-8-cyclohexyl-1H-pyrazino( 3,2,1-j,k)carbazole hydrochloride were obtained. The characteristics of the thus-prepared compound were similar to those described in Example 1 hereinbefore.

Example 5

To 100 ml of toluene and 100 ml of a 30% solution of caustic soda 20 g (0.0735 g/mol) of a 98% 6-cyclohexyl-3,4-dihydrocarbazol-1(2H)-one were added and after stirring for 5-10 minutes 27.75 g (0.0899 g/mol) 98% β -(N,N-dibenzylamino )-ethylchloride hydrochloride and 1 ml of polyethylene glycol were added thereto.

There were thus obtained 34.0 g (94.52%) of 6-cyclohexyl-9 β-(N,N-dibenzylamino)ethyl-3,4-dihydrocarbazol-1(2H)-one melting at 103-104°C which were used for the preparation of the desired product in a manner similar to that described in Example 1 hereinbefore.

Example 6

To 100 ml of toluene and 100 ml of a 30% solution of caustic soda 20 g of a 98% (0.0735 g/mol) 6-cyclohexyl-3,4-dihydrocarbazol-1(2H)-one were added and after stirring for 5-10 minutes 27.75 g (0.089 g/mol) of a 96% β-(N,N-dibenzylamino)ethylchloride hydrochloride and 1.5 ml of a 50% solution of dimethyloctylbenzylammmonium chloride were added thereto. The reaction was carried out in a manner similar to that described in Example 1.

There were thus obtained 34.69 g (96.43%) of 6-cyclohexyl-9 β -(N,N-dibenzylamino)ethyl-3,4-dihydrocarbazol-1(2H)-onemelting at 103-104°C which were employed for the preparation of the desired product in a manner similar to that described in Example 1 hereinbefore.

Example 7

To 200 ml of chlorobenzene and 90 ml of a 30% aqueous solution of caustic soda 40.92 g (0.15 g/mol) of a 98% 6-cyclohexyl-3,4-dihydrocarbazol-1(2H)-one (II) were added along with 53.59 g (0.18 g/mol) of a 99.5% β-(N,N-dibenzylamino)ethylchloride hydrochloride, 1.5 ml of benzyl chloride and 1.5 ml of triethylamine. The reaction was conducted in a manner similar to that of Example 1 hereinbefore, except that the isolation of the obtained product (compound IV) was carried out from methanol.

There were thus obtained 72 g of 6-cyclohexyl-9 β-(N,N-dibenzylamino)-ethyl-3,4-dihydrocarbazol-1-(2H)-one (IV) (or 96.26% by mass as calculated for the starting compound II).

A suspension of 20.33 g (0.0408 g/mol) of 6-cyclohexyl-9 β (N,N-dibenzylamino)ethyl-3,4-dihydrocarbazol-1(2H)-one in 150 ml of methanol was hydrogenated with hydrogen in the presence of 1.4 g of palladium bisacetylacetonate and 4 g of activated carbon under a pressure of 10-20 atm and at the temperature of 60°C.

On completion of the reaction the catalyst was filtered-off, washed with methanol and the filtrate was treated with a concentrated hydrochloric acid to a pH of 1-2. The formed precipitate after cooling was filtered-off, washed with acetone and dried.

There were thus obtained 11.82 g (84.35% by mass as calculated for the starting compound II) of a commercial desire product. After recrystallization (from aqueous methanol) 10.74 g (76.65% by mass as calculated for the starting compound II) of 2,3,3a,4,5,6-hexahydro-8-cyclohexyl-1H-pyrazino-(3,2,1-j,k)-carbazole hydrochloride were obtained. The characteristics of the thus-prepared compound were similar to those described in Example 1 hereinbefore.

Industrial Applicability

The compound according to the present invention exhibits an antidepressant activity and can be useful in medicine as an active principle of a pharmaceutical preparation for the treatment of depressive states of a different etiology.

**Claims**

**1.** 2,3,3a,4,5,6-hexahydro-8-cyclohexyl-1H-pyrazino(3,2,1-j,k)carbazole hydrochloride of the formula:

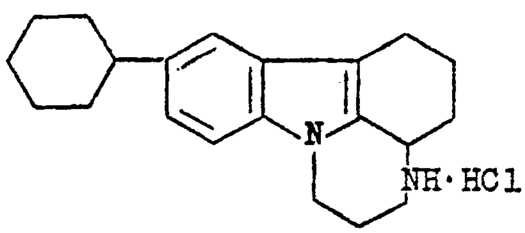

**2.** A method for preparing 2,3,3a,4,5,6-hexahydro-8-cyclohexyl-1H-pyrazino(3,2,1-j,k)carbazole hydrochloride according to Claim 1, **characterized** by alkylating 6-cyclohexyl-3,4-dihydrocarbazol-1(2H)-one with β-(N,N-dibenzylamino )ethylchloride hydrochloride in a two-phase system: an aromatic hydrocarbon - a 30-40% aqueous solution of an alkali in the presence of a catalyst of an interphase transfer selected from a tetra-alkyl-, or trialkylbenzyl-, or a trialkyl(alkylbenzyl )ammonium halide, or a mixture of benzyl chloride and triethylamine, or dimethylformamide, or polyethyleneglycol, at a temperature within the range of from 85 to 110°C, followed by hydrogenation of the resulting 6-cyclohexyl-9 β -(N,N-dibenzylamino)ethyl-3,4-dihydrocarbazol-1(2H)-oneover a palladium catalyst supported on a carrier in a medium of a lower aliphatic alcohol at a temperature of from 50 to 70°C under a pressure of from 1 to 30 atm, with subsequent conversion of the resulting 2,3, 3a,4,5,6-hexahydro-8-cyclohexyl-1H-pyrazino-(3,2,1-j,k)-carbazole into hydrochloride and isolation of the latter.

**3.** A method according to Claim 2, **characterized** in that the palladium catalyst comprises palladium bisacetylacetonate or palladium hydroxide and the carrier comprises carbon of asbestos.

**4.** A method according to Claim 2, **characterized** in that the palladium catalyst palladium comprises bisacetylacetonate supported on carbon, the process being conducted at a temperature within the range of from 50 to 60°C under a pressure ranging from 10 to 20 atm.

**5.** A pharmaceutical preparation of an antidepressant effect comprising an active principle and a pharaceutically acceptable carrier, **characterized** in that the active principle comprises 2,3,3a,4,5,6-hexahydro-8-cyclohexyl-1H-pyrazino(3,2,1-j ,k)carbazole hydrochloride according to Claim 1.

6. A pharmaceutical preparation according to Claim 5 in a tablet form, **characterized** in that it contains the active principle in the amount of 25 mg per tablet.

# INTERNATIONAL SEARCH REPORT

International Application No PCT/SU 89/00302

**I. CLASSIFICATION OF SUBJECT MATTER** (if several classification symbols apply, indicate all)

According to International Patent Classification (IPC) or to both National Classification and IPC

$IPC^5$ A 61 K 31/495, 9/20 C 07 D 487/04, 209/86

**II. FIELDS SEARCHED**

Minimum Documentation Searched

| Classification System | Classification Symbols |
|---|---|
| $IPC^4$ | A 61 K 31/495, 31/435, 9/20; 31/40<br>C 07 D 209/86, 487/04, 471/06 |

Documentation Searched other than Minimum Documentation to the Extent that such Documents are Included in the Fields Searched

**III. DOCUMENTS CONSIDERED TO BE RELEVANT**

| Category * | Citation of Document, with indication, where appropriate, of the relevant passages | Relevant to Claim No. |
|---|---|---|
| A | US, A, 4400383 (JONATHAN R.T. DAVIDSON, ANTHONY T. DREN), 23 August 1983 (23.08.83) column 7, lines 67,68, column 8, lines 1-66; column 12, example 10 | 5,6 |
| A | SU, A3, 906380 ("KASELLA AG") 17 February 1982 (17.02.82), column 7, lines 29-54, column 8, lines 9-12 | 5,6 |
| A | US A, 3959470 (MIKHAIL DAVIDOVICH MASHKOVSKY et al.) 25 May 1976 (25.05.76) column 2, lines 22-66, column 2, lines 3-54<br>DE 2356091 A<br>FR 2207727 A<br>GB 1444980 A | 5,6 |

./...

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| 26 June 1990 (26.06.90) | 30 July 1990 (30.07.90) |
| International Searching Authority | Signature of Authorized Officer |
| ISA/SU | |

III. DOCUMENTS CONSIDERED TO BE RELEVANT (CONTINUED FROM THE SECOND SHEET)

| Category* | Citation of Document, with indication, where appropriate, of the relevant passages | Relevant to Claim No |
|---|---|---|
| A | SU, A1, 276061 (Vsesojuzny nauchno-issledovatelsky khimiko-farmatsevtichesky institut im. Sergo Ordzhonikidze), 25 August 1976 (25.08.76) | 1-4 |
| A | SU, A5, 581654 (Vsesojuzny nauchno-issledovatelsky khimiko-farmatsevtichesky institut im. Sergo Ordzhonikidze), 15 November 1976 (15.11.76) the claims | 1-4 |
| A | GB, B, 1340529 (Vsesojuzny nauchno-issledovatelsky khimiko-farmatsevtichesky institut imeni Sergo Ordzhonikidze) 12 December 1973 (12.12.73) , the claims | 1-4 |